# EUROPEAN PATENT SPECIFICATION

(11) **EP 4 282 852 B1**
(45) Date of publication and mention of the grant of the patent: **17.09.2025**
(21) Application number: 22742913.1
(22) Date of filing: 25.01.2022
(51) Int. Cl.: C07C 7/20, C07C 15/06, C07C 17/12

(54) **METHOD FOR CHLORINATING AROMATIC COMPOUND**
VERFAHREN ZUR CHLORIERUNG EINER AROMATISCHEN VERBINDUNG
PROCÉDÉ DE CHLORATION D'UN COMPOSÉ AROMATIQUE

(30) Priority: 25.01.2021 KR 20210010151
(43) Date of publication of application: 29.11.2023
(73) Proprietor: Hanwha Solutions Corporation, Jung-gu Seoul 04541 (KR)
(72) Inventor: JANG, Namjin, Daejeon 34128 (KR); CHOI, Ji Hye, Daejeon 34114 (KR); PARK, Jinho, Daejeon 34128 (KR)
(74) Representative: Berggren Oy
(86) International application number: PCT/KR2022/001268
(87) International publication number: WO 2022/158937

(56) References cited:
- CN-A- 109 970 507
- JP-A- 2006 160 611
- JP-A- 2010 120 934
- JP-B2- 5 658 865
- JP-B2- 5 669 362
- KR-B1- 101 759 512
- KR-B1- 101 759 512

## Description

### [Technical Field]

The present invention relates to a method for chlorinating an aromatic compound, which is toluene and more specifically, tc a method for chlorinating toluene which may prevent a reactivity decrease phenomenon caused by foaming and prevent counterflow of a fluid.

### [Background Art]

Chlorobenzene or chlorotoluene is a material used in various industrial fields, and is formed by a chlorination reaction of an aromatic compound such as benzene and toluene. As a specific example, monochlorotoluene such as o-chlorotoluene and p-chlorotoluene may be prepared by reacting toluene and chlorine in the presence of a catalyst such as FeCl₃. Specifically, when toluene and chlorine are reacted in the presence of a catalyst, a chlorine atom is substituted to produce monochlorotoluene such as o-chlorotoluene and p-chlorotoluene.

Conventionally, when an aromatic compound is chlorinated to prepare chlorobenzene or chlorotoluene as described above, overchlorinated by-products such as dichlorobenzene or dichlorotoluene may be produced depending on a supply ratio of the aromatic compound and the chlorine gas. In order to suppress the production of the by-product as much as possible, in the common production method, benzene or toluene is used in an excessive amount as compared with chlorine, and a large amount of unreacted benzene or toluene, which is mixed with the product after the reaction, is recovered and used again as a reactant.

However, like the common production method, by-products such as dichlorobenzene or dichlorotoluene are produced in a significant amount even with the use of a large amount of benzene or toluene as compared with chlorine, and thus, selectivity and yield are not increased beyond a certain level.

In order to solve the problem of the prior art as such, in Korean Patent Registration No. 10-1759512 which was previously filed by the present applicant, toluene and chlorine are reacted in one reactor having a multistage structure including a reaction unit and a cooling unit to improve the selectivity of chlorotoluene and p-chlorotoluene, thereby increasing the reaction yield.

However, in the reactor having a multistage structure, as the chlorination reaction occurs, a gas ratio is increased over time, and a residence time of a chlorine gas is decreased toward the upper portion and it is difficult to control a linear velocity of gas in a reactor. Specifically, as a reaction time elapses, the linear velocity of gas in the reactor is increased, and above a certain linear velocity, foam is produced to decrease reactivity and rather decrease a yield.

Thus, the present inventors studied in depth an efficient method of increasing a product yield by maintaining high reactivity even when a chlorination reaction time is elapsed.

### [Disclosure]

### [Technical Problem]

An object of the present invention is to provide a method for chlorinating toluene which may prevent a reactivity decrease phenomenon caused by foaming and prevent counterflow of a liquid in a reactor.

### [Technical Solution]

In one general aspect, a method for chlorinating toluene in a plurality of column-type reactors connected in series, introducing a reaction product produced in a former reactor to a latter reactor, introducing a chlorine gas to a low portion of each reactor in the same amount to perform a chlorination reaction in each reactor, and discharging a hydrogen chloride gas produced in each of the reactors from each reactor.

In the method for chlorinating toluene according to an exemplary embodiment of the present invention, the reaction product produced in the former reactor may be cooled by a heat exchanger disposed between the adjacent reactors and be introduced to the low portion of the latter reactor quantitatively.

In the method for chlorinating toluene according to an exemplary embodiment of the present invention, the hydrogen chloride gas may be further discharged from the reaction product produced in the former reactor by a buffer drum and then the reaction product may be supplied to the latter reactor.

In the method for chlorinating toluene according to an exemplary embodiment of the present invention, the chlorine gas may be introduced to the low portion in the reactor through a gas sparger which is disposed in each reactor and has a plurality of holes formed thereon.

In the method for chlorinating toluene according to an exemplary embodiment of the present invention, a diameter of the hole of the gas sparger may be 1 to 5 mm.

In the method for chlorinating toluene according to an exemplary embodiment of the present invention, the chlorine gas may be introduced at a linear velocity of 5 to 10 m/sec through the gas sparger hole.

In the method for chlorinating toluene according to an exemplary embodiment of the present invention, the aromatic compound and the chlorine gas may be introduced at 1:1/16 to 1/8 based on the total moles.

In the method for chlorinating toluene according to an exemplary embodiment of the present invention, a conversion rate in each reactor may be 1 to 15%.

In the method for chlorinating toluene according to an exemplary embodiment of the present invention, the aromatic compound is introduced to the low portion in the reactor through a raw material supply nozzle which is disposed in a lower portion than the gas sparger in the reactor, and the aromatic compound may be introduced at a linear velocity of 10 to 40 m/sec through the raw material supply nozzle.

In the method for chlorinating toluene according to an exemplary embodiment of the present invention, the linear velocity of gas in each reactor may be 7 cm/sec or less.

In the method for chlorinating toluene according to an exemplary embodiment of the present invention, the reaction product may be cooled to a temperature of 0°C or higher and 25°C or by the heat exchanger.

In the method for chlorinating toluene according to an exemplary embodiment of the present invention, a bubble diameter of the chlorine gas introduced to each reactor may be 0.1 mm or more and 5 mm or less.

In the method for chlorinating toluene according to an exemplary embodiment of the present invention, the reaction of each reactor may be performed in the presence of a main catalyst and a cocatalyst.

In the method for chlorinating toluene according to an exemplary embodiment of the present invention, the main catalyst may be at least one selected from the group consisting of FeCl₃, ferrocene, PtO₂, SbCl₃, and Fe.

In the method for chlorinating toluene according to an exemplary embodiment of the present invention, the cocatalyst may be at least one selected from the group consisting of S₂Cl₂, thianthrene, diphenylselenide, tetrachlorophenoxathin, dichlorothiantrene, tetrachlorothiantrene, and polychlorothiantrene.

In the method for chlorinating toluene according to an exemplary embodiment of the present invention, contents of the main catalyst and the cocatalyst (main catalyst : cocatalyst) may be 1:0.59 or more and 1:0.76 or less, based on a mole ratio.

### [Advantageous Effects]

In the method for reacting toluene according to the present invention, the same amount of chlorine gas is supplied to a plurality of reactors arranged in series and a reaction product produced in a former reactor is supplied to a latter reactor, thereby preventing foaming and a decrease in chlorination reactivity caused by an increase in gas volume over a reaction time, and thus, increasing a reaction yield of the product to be prepared.

In addition, a conversion rate of the reacted product is improved to decrease formation of wastes such as chlorine as an unreacted reactant and hydrogen chloride (HCl) as a reaction by-product, and thus, an environmentally friendly and economical method for reacting an aromatic compound is provided.

### [Description of Drawings]

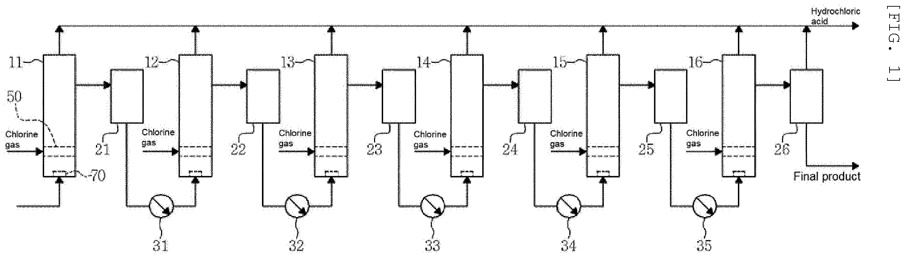

FIG. 1 is an exemplary schematic diagram of a reaction device which is used in a method for chlorinating an aromatic compound according to an exemplary embodiment of the present invention.

### [Best Mode]

Unless otherwise defined herein, all terms used in the present specification (including technical and scientific terms) may have the meaning that is commonly understood by those skilled in the art. In the entire specification, unless otherwise particularly described, a part "comprising" a constituent element will be understood to imply further inclusion of other constituent elements rather than the exclusion of any other constituent elements. In addition, unless otherwise particularly described, a singular form includes a plural form herein.

The terms "about", "substantially", and the like used in the present specification are used in the meaning of the numerical value or in the meaning close to the numerical value when unique manufacture and material allowable errors are suggested in the mentioned meaning, and are used for preventing the disclosure mentioning a correct or absolute numerical value for better understanding of the present application from being unfairly used by an unconscionable infringer.

In the present specification, the term "combination (s) thereof" included in the expression in a Markush format means a mixture of combination of one or more selected from the group consisting of constituents described in the expression in a Markush format, and means inclusion of one or more selected from the group consisting of the constituents.

Conventionally, when an aromatic compound is chlorinated to prepare chlorobenzene or chlorotoluene, overchlorinated by-products such as dichlorobenzene or dichlorotoluene were produced depending on a supply ratio of the aromatic compound and chlorine gas. Thus, a reaction yield was intended to be increased by reacting toluene and chlorine in one reactor having a multistage structure including a reaction unit and a cooling unit to improve selectivity of o-chlorotoluene and p-chlorotoluene, but since a chlorination reaction occurs in the reactor having a multistage structure, a gas ratio is increased over time, so that a residence time of a chlorine gas is decreased toward the upper portion and it is difficult to control a linear velocity of gas in the reactor. Thus, as the reaction time elapses, a linear velocity exceeds a certain level to cause foam, thereby decreasing a yield.

In the present invention, a plurality of column-type reactors are connected in series so that a reaction product produced in a former reactor is introduced to a latter reactor, chlorine gas is introduced to a low portion of each reactor in the same amount to perform a chlorination reaction in each reactor, and a hydrogen chloride gas produced in each reactor is discharged from each reactor. Thus, foaming and a decrease in chlorination reactivity caused by an increase in gas volume due to the elapse of the reaction time are prevented, thereby increasing a reaction yield of the product to be prepared.

In addition, a conversion rate of the product is improved to decrease occurrence of wastes such as chlorine as an unreacted reactant and hydrogen chloride (HCl) as a reaction by-product, thereby providing an environmentally friendly and economical method for reacting an aromatic compound.

Specifically, in the plurality of reactors arranged in series, a reaction product produced in a foremost reactor which is disposed at the forefront and in which a chlorine gas is first supplied to a raw material including an aromatic compound may be supplied to a latter reactor adjacent thereto, and sequentially, be passed through each reactor to be supplied to a reactor disposed at the last stage.

In each reactor, the chlorine gas is injected into the reaction product produced in a former stage reaction, that is, the raw material including toluene to produce a product by the chlorination reaction of toluene and the chorine gas.

In an exemplary embodiment of the present invention, the aromatic compound is toluene, and the product may be a chlorinated product thereof, but they are not limited thereto. In a specific example, the chlorine gas may be supplied to toluene which is the aromatic compound to produce o-chlorotoluene and p-chlorotoluene. More specifically, an excessive amount of toluene may be supplied to the inside of each reactor, in which "excessive amount" means, as a specific example, an environment in which toluene is present in an amount greater than a stoichiometric reaction equivalence ratio (1:1) of toluene and chlorine gas which are the reactants of the following Reaction Formula 1. Thus, in the environment, the chlorine gas to be injected may be all reacted with toluene.

The step of supplying the chlorine gas to toluene to produce o-chlorotoluene and p-chlorotoluene may be performed by a reaction represented by the following Reaction Formula 1:

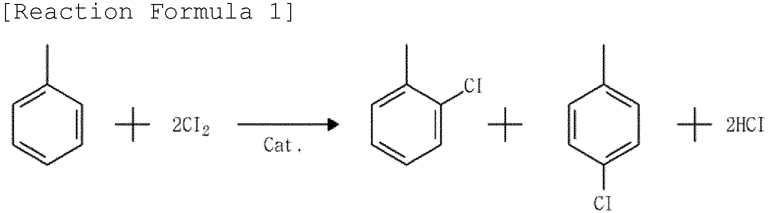

As such, since the hydrogen chloride gas is included in the final product, the chlorination reaction of toluene of the present invention has an increased gas ratio as the reaction proceeds, and foam may occur as the linear velocity of gas in the reactor is increased. Thus, the yield of the final product may be affected.

In the present invention, since the hydrogen chloride gas produced in each reactor is discharged to the upper portion, when the reaction product is supplied from the former reactor to the latter stage, the hydrogen chloride gas is removed from the reaction product. In addition to the hydrogen chloride gas, unreacted chlorine gas may be discharged, of course.

The present invention as such may prevent foaming caused by a linear velocity increase of gas due to an increase in gas volume in the reactor as the reaction time elapses, and since the constant linear velocity of gas in each reactor may be maintained, it is easy to control the linear velocity and it may be advantageous to design a production preparation process and maintain the process. Furthermore, occurrence of counterflow when a liquid such as a raw material is supplied due to an increase in gas volume inside the reactor may be prevented.

In an exemplary embodiment of the present invention, a reaction product produced in the former reactor by a buffer drum disposed between adjacent reactors may be supplied to the latter reactor after further discharging a hydrogen chloride gas. As the buffer drum, a buffer drum which is used in removing hydrogen chloride gas in the art may be applied, and an accommodation space which may accommodate the reaction product from which the hydrogen chloride gas has been first discharged in the former reactor is formed, and the hydrogen chloride gas is secondarily discharged to remove the hydrogen chloride gas included in the reaction product as much as possible. In the buffer drum, the reaction product from which the hydrogen chloride gas is secondarily removed may be supplied to the latter reactor. As such, when the reaction product in the former reactor is supplied through the buffer drum to the latter reactor, the hydrogen chloride gas in the reaction product may be secondarily removed, and thus, a high-purity product may be produced and also the linear velocity of the gas in the reactor may be more easily adjusted.

Meanwhile, since the reaction between toluene and the chlorine gas is exothermic, the temperature of the reaction solution is raised as the reaction proceeds. Thus, as the more chlorine gas is introduced to excessive toluene, the more the reaction proceeds and the higher the temperature of the reaction solution rises. Since the selectivity of the chlorinated reaction product of the aromatic compound such as o-chlorotoluene and p-chlorotoluene tends to be decreased as the reaction temperature rises, the selectivity may be decreased when the reaction proceeds too much.

Thus, in an exemplary embodiment of the present invention, the reaction product produced in the former reactor may be cooled by the heat exchanger disposed between adjacent reactors and introduced to the low portion of the latter reactor quantitatively. The cooling may be performed at a temperature of 0°C or higher and about 50°C or lower, specifically 5°C or higher and 30°C or lower, but the cooling may proceed without limitation as long as chlorine is not liquefied. Specifically, depending on the supply pressure of the chlorine gas, the temperature may be 25°C at 7.81 bara, and when the supply pressure of chlorine is 5.07 bara, the temperature may be 10°C. When the supply pressure of the chlorine gas is 3.7 bara, the cooling may be performed to 0°C. However, in order to lower the temperature of the chlorine gas, a refrigerant at a temperature lower than the temperature to perform the cooling is needed, and cooling more than needed causes excessive investment. Therefore, considering chlorine liquefaction temperature, operating cost, and the like depending on operating pressure, the above range may be preferred. However, the present invention is not limited thereto.

In an exemplary embodiment of the present invention, the injection of the chlorine gas may be performed through a gas sparger which is disposed in each reactor and has a plurality of holes formed thereon. Specifically, the gas sparger is disposed in the low portion inside the reactor and introduces the chlorine gas to the low portion inside the reactor. The gas sparger is not limited and any gas sparger may be applied as long as it is used in the art. A diameter of the hole (nozzle) of the gas sparger from which the chlorine gas is sprayed may be 0.5 mm to 5 mm, specifically 1 to 3 mm, but is not limited thereto. However, the linear velocity of the chlorine gas may be easily set in the range.

The chlorine gas may be supplied in the same amount in each reactor, and may be introduced (injected) at a linear velocity in the gas sparger hole of 1 to 20 m/sec, specifically 5 to 10 m/sec through the gas sparger. Since the chlorine gas is injected at the linear velocity as such, the linear velocity of 1 to 7 cm/sec may be shown in the reactor, and the linear velocity is lowered toward the upper portion in the column-type reactor, thereby solving the problems of a decrease in a reaction rate and foaming due to agglomeration of gas particles.

The amount of the chlorine gas in the reactor may be less than 1/8, based on the total moles of excessive toluene in the raw material (reaction solution) including the aromatic compound. More specifically, it may be more than 1/16 and less than 1/8 or 1/14 or more and 1/10 or less, but is not limited thereto. However, by introducing the chlorine gas in the above range, the reaction between toluene and chlorine may be performed with a high selectivity throughout the process.

A bubble diameter of the chlorine gas to be injected may be 0.1 mm or more and 5 mm or less. It is preferred that the chlorine gas to be injected into the reaction solution has a uniform size distribution. When the bubble of the chlorine gas is too small, the reactivity of a side reaction product may be increased to decrease the selectivity. When the bubble diameter of the chlorine gas is too large, the reactivity of the chlorine gas is decreased so that the size of the reactor should be large, and thus, process costs such as investment and operating costs may be unnecessarily increased.

In an exemplary embodiment of the present invention, the injection of toluene may be performed through a raw material supply nozzle disposed in the lower portion than the gas sparger in each reactor. The raw material including the aromatic compound may be supplied to the lower portion than the chlorine gas inside the reactor through the raw material supply nozzle disposed in the lower portion than the gas sparger. A diameter of the hole (nozzle) of the raw material supply nozzle from which the aromatic compound is sprayed may be 1 mm to 9 mm, specifically 1 to 5 mm, but is not limited thereto. Toluene may be introduced at a linear velocity of 10 to 60 m/sec, specifically 10 to 40 m/sec through the raw material supply nozzle, but is not limited thereto. However, within the range, the linear velocity of a liquid may be shown in the reactor described later.

In the reactor, an average liquid linear velocity may be 0.1 cm/s or more and 100 cm/s or less. Within the range, the residence time of the reactant in the reactor is adjusted, so that excellent reactivity may be shown.

Specifically, as a method for increasing the liquid linear velocity inside the reactor for effective heat removal (cooling), when the flow inside the reactor is improved, back mixing occurs by counterflow to lower the selectivity. In addition, when the linear velocity of the chlorine gas is increased by the increase in a liquid linear velocity, the conversion rate of the chlorine gas is less than 100%, and the consumed amount of chlorine gas is increased. When the conversion rate of chlorine is low, it is difficult to separate the hydrogen chloride gas and the chlorine gas which are reaction products, and thus, the chlorine gas is treated as a large amount of wastes.

Since toluene and the chlorine gas are supplied in the above ranges, in the present invention, gases in the reactor have constant linear velocities, and a decrease in selectivity and waste of chlorine gas due to the back mixing may be prevented. Specifically, the linear velocity of gas inside each reactor may be 10 cm/sec or less, more specifically 4 cm/sec or less. Since foaming is significantly suppressed in the range, the product may be produced at a high yield. Herein, the gas may include hydrogen chloride gas produced by the reaction as well as chlorine gas in the reactor.

In an exemplary embodiment of the present invention, the chlorine gas may be introduced at 5 to 10 L/min, based on the aromatic compound introduced at 650 g/min in the reactor having an inner diameter of 13 cm and a height of 600 cm. The conversion rate in each reactor is 1 to 15% in the range, and an excellent conversion rate may be shown.

In an exemplary embodiment of the present invention, the reaction may be performed in the presence of a main catalyst and a cocatalyst. Specifically, the main catalyst may be one or more selected from the group consisting of FeCl₃, ferrocene, PtO₂, SbCl₃, and Fe. The cocatalyst may be one or more selected from the group consisting of S₂Cl₂, thianthrene, diphenylselenide, tetrachlorophenoxathin, dichlorothiantrene, tetrachlorothiantrene, and polychlorothiantrene.

In addition, contents of the main catalyst and the cocatalyst (main catalyst : cocatalyst) may be 1:0.59 or more and 1:0.76 or less, based on a mole ratio. This is the condition to improve the selectivities of o-chlorotoluene and p-chlorotoluene to about 99.5% or more, as recognized by referring Korean Patent Appln. No. 10-2016-0069830 which is the preceding invention of the present inventors.

Though the contents of the main catalyst and the cocatalyst of the present invention are not limited to the range, when the technology is applied to the present invention, the effect of improving selectivity may be further improved.

In the chlorination method according to an exemplary embodiment of the present invention, the product may be separated and recovered after the reaction is completed in the last reactor. In addition to the product to be desired, an unreacted aromatic compound, excessive chlorinated by-products, the main catalyst, and the cocatalyst remain by the reaction of toluene and the chlorine gas, and thus, separation for reuse thereof is required. Various methods known in the art may be adopted for the separation.

Hereinafter, a specific example of performing the reaction method according to an exemplary embodiment of the present invention will be described as the case performed in a plurality of reactors connected in series, referring to FIG. 1.

First, six identical column-type reactors are arranged in a row parallel to the ground, and a buffer drum and a heat exchanger are disposed, respectively, between the reactors. Herein, as illustrated in the drawing, the buffer drum may be further provided in the latter stage of the last reactor.

Further, a gas sparger disposed in the reactor disposed in the forefront stage and a raw material including a chlorine gas and an excessive amount of toluene through a raw material supply nozzle are introduced, respectively.

The chlorination method of the present invention may be performed by the chlorination reaction in each reactor, sequentially through the reactors from the foremost reactor to the last reactor.

Hereinafter, description will be provided assuming that a reactor disposed in the forefront stage is referred to as a first reactor 11, a reactor disposed in the latter stage adjacent thereto is referred to as a second reactor 12, reactors disposed in the latter stages adjacent thereto are sequentially referred to as first to sixth reactors 11, 12, 13, 14, 15, and 16, to the last stage, and in this manner, buffer drums are referred to as first to sixth buffer drums 21, 22, 23, 24, 25, and 16 and heat exchangers are referred to as first to fifth heat exchangers 31, 32, 33, 34, and 35.

A reaction product produced in the first reactor 11 may be subjected to a first removal of hydrogen chloride gas from the first reactor 11, followed by a second removal of hydrogen chloride gas through the first buffer drum 21. Thereafter, it may be supplied to the first heat exchanger 31, cooled through the first heat exchanger 31, and supplied to the second reactor 12. In this manner, it may be passed through the second to fifth reactors 12, 13, 14, and 15, the second to fifth buffer drums 22, 23, 24, and 25, and the second to fifth heat exchangers 32, 33, 34, and 35, and finally supplied to the sixth reactor 16. The reaction product which finally completed the reaction in the sixth reactor 16 may be passed through the sixth buffer drum 26 to remove hydrogen chloride.

To the first to sixth reactors 11, 12, 13, 14, 15, and 16, each chlorine gas is identically supplied, and in each reactor, the introduced chlorine gas and the raw material including the aromatic compound or the reaction produce supplied from the former reactor are reacted to produce a chlorinated product. The reaction product supplied to each reactor is passed through the first to fifth heat exchangers 31, 32, 33, 34, and 35 to be cooled to 0°C or higher and 25°C or lower. The reason for cooling to the temperature range is as described above.

Hereinafter, the preferred examples and the comparative examples of the present invention will be described. However, the following examples are only a preferred exemplary embodiment of the present invention, and the present invention is not limited thereto.

### (Example 1)

Toluene and a chlorine gas were introduced into a reactor having an inner diameter of 13 cm and a height of 600 cm, in which the linear velocity of gas in the reactor was adjusted by adjusting the chlorine gas to be introduced at a linear velocity of 5 to 10 m/sec through a gas sparger hole and the raw material including toluene to be introduced at 10 to 40 m/sec through a raw material supply nozzle. At this time, a mole ratio between toluene and chlorine was 2:1, FeCl₃ was used as a catalyst, S₂Cl₂ was used as a cocatalyst, and concentrations of F₃Cl₃ and S₂Cl₂ were 300 ppm and 150 ppm, respectively.

Thereafter, a gas-hold up which is a gas volume fraction in the total volume of gas-liquid phase was measured, a surface area was measured by the bubble size and the gas-hold up in the reactor, and it was determined whether foam was formed, for the reactor. The size of a chlorine bubble in the nozzle of the sparger was injected as 2 mm. The results are shown in the following Table 1.

**[Table 1]**

| Linear velocity (cm/sec) | Gas-hold up | Surface area per unit volume (m²/m³) |
|---|---|---|
| 1 | 0.041 | 120 |
| 2 | 0.085 | 254 |
| 3 | 0.131 | 393 |
| 4 | 0.179 | 536 |
| 5 | 0.227 | 682 |
| 6 | 0.227 | 831 |
| 7 | 0.327 | 981 |

Referring to Table 1, it was confirmed that foam was hardly formed at a linear velocity of 4 or less.

### (Example 2)

To a reactor having an inner diameter of 13 cm and a height of 600 cm, toluene was introduced at 650 g/min and a chlorine gas was introduced at 5 L/min. Chlorine was supplied at 7 m/sec through a gas sparger hole and toluene was supplied at 20 m/sec through a liquid sparger hole.

The linear velocity of gas in the reactor was 1.77 cm/sec.

At this time, a toluene conversion rate was calculated by the following Calculation Formula 1: Toluene conversion rate = (supply amount - toluene after reaction) / supply amount

In Example 2, the toluene conversion rate was confirmed to be 2.7% which is the theoretical conversion rate. Accordingly, it was confirmed that it is very easy to design a chlorination reaction in the present invention.

## Claims

1. A method for chlorinating an aromatic compound which is toluene, the method comprising:
in a plurality column-type reactors connected in series, introducing a reaction product produced in a former reactor to a latter reactor,
introducing a chlorine gas to a low portion of each reactor in the same amount to perform a chlorination reaction in each reactor, and
discharging a hydrogen chloride gas produced in each of the reactors from each reactor.

2. The method of claim 1, wherein the reaction product produced in the former reactor is cooled by a heat exchanger disposed between the adjacent reactors and introduced to a low portion of the latter reactor quantitatively.

3. The method of claim 1, wherein the hydrogen chloride gas is further discharged from the reaction product produced in the former reactor by a buffer drum disposed between the adjacent reactors and then the reaction product is supplied to the latter reactor.

4. The method of claim 1, wherein the chlorine gas is introduced to a low portion in the reactor through a gas sparger which is disposed in each reactor and has a plurality of holes formed thereon.

5. The method of claim 4, wherein a diameter of the hole of the gas sparger is 1 to 5 mm.

6. The method of claim 1, wherein the chlorine gas is introduced at a linear velocity of 5 to 10 m/sec through the gas sparger hole.

7. The method of claim 1, wherein the aromatic compound and the chlorine gas are introduced at 1:1/16 to 1/8 based on the total moles.

8. The method of claim 7, wherein a conversion rate in each reactor is 1 to 15%.

9. The method of claim 4, wherein the aromatic compound is introduced to the lower portion of the reactor through a liquid sparger which is disposed in a lower portion than the gas sparger in the reactor and has a plurality of holes of raw material supply nozzle formed thereon, and
the aromatic compound is introduced at a linear velocity of 10 to 40 m/sec through the raw material supply nozzle.

10. The method of claim 1, wherein the linear velocity of gas in each reactor is 47 cm/sec or less.

11. The method of claim 2, wherein the reaction product is cooled to a temperature of 0°C or higher and 25°C or lower by the heat exchanger.

12. The method of claim 10, wherein a bubble diameter of the chlorine gas introduced to each of the reactors is 0.1 mm or more and 5 mm or less.

13. The method of claim 1, wherein the reaction of each of the reactors is performed in the presence of a main catalyst and a cocatalyst, preferably
wherein the main catalyst is at least one selected from the group consisting of FeCl₃, ferrocene, PtO₂, SbCl₃, and Fe, and/or
the cocatalyst is at least one selected from the group consisting of S₂Cl₂, thianthrene, diphenylselenide, tetrachlorophenoxathin, dichlorothiantrene, tetrachlorothiantrene, and polychlorothiantrene.

14. The method of claim 13, wherein contents of the main catalyst and the cocatalyst (main catalyst : cocatalyst) is 1:0.59 or more and 1:0.76 or less based on a mole ratio.

## Patentansprüche

1. Verfahren zur Chlorierung einer aromatischen Verbindung, welche Toluol ist,
wobei das Verfahren umfasst:
in einer Mehrzahl von in Reihe geschalteten Reaktoren vom Säulentyp, das Einführen eines in einem ersten Reaktor erzeugten Reaktionsprodukts in einen zweiten Reaktor,
das Einführen eines Chlorgases in einen unteren Teil jedes Reaktors in der gleichen Menge, um eine Chlorierungsreaktion in jedem Reaktor durchzuführen, und
das Abführen eines in jedem der Reaktoren erzeugten Chlorwasserstoffgases aus jedem Reaktor.

2. Verfahren nach Anspruch 1, wobei das im ersten Reaktor erzeugte Reaktionsprodukt durch einen zwischen den benachbarten Reaktoren angeordneten Wärmetauscher gekühlt und in einen unteren Teil des zweiten Reaktors quantitativ eingeführt wird.

3. Verfahren nach Anspruch 1, wobei das Chlorwasserstoffgas aus dem im ersten Reaktor erzeugten Reaktionsprodukt durch eine zwischen den benachbarten Reaktoren angeordnete Puffertrommel weiter abgeführt und das Reaktionsprodukt dann dem zweiten Reaktor zugeführt wird.

4. Verfahren nach Anspruch 1, wobei das Chlorgas durch einen Gasverteiler, der in jedem Reaktor angeordnet ist und an dem eine Mehrzahl von Löchern ausgebildet ist, in einen unteren Bereich des Reaktors eingeführt wird.

5. Verfahren nach Anspruch 4, wobei ein Durchmesser des Lochs des Gasverteilers 1 bis 5 mm beträgt.

6. Verfahren nach Anspruch 1, wobei das Chlorgas mit einer linearen Geschwindigkeit von 5 bis 10 m/Sek durch das Gasverteilerloch eingeführt wird.

7. Verfahren nach Anspruch 1, wobei die aromatische Verbindung und das Chlorgas im Verhältnis 1:1/16 bis 1/8, bezogen auf die Gesamtmolzahl, eingeführt werden.

8. Verfahren nach Anspruch 7, wobei die Umsetzungsrate in jedem Reaktor 1 bis 15 % beträgt.

9. Verfahren nach Anspruch 4, wobei die aromatische Verbindung in den unteren Teil des Reaktors durch einen Flüssigkeitsverteiler eingeführt wird, der in einem niedrigeren Teil als der Gasverteiler in dem Reaktor angeordnet ist und eine Mehrzahl von daran ausgebildeten Löchern einer Rohstoffzufuhrdüse aufweist, und
die aromatische Verbindung mit einer linearen Geschwindigkeit von 10 bis 40 m/Sek durch die Rohstoffzufuhrdüse eingeführt wird.

10. Verfahren nach Anspruch 1, wobei die lineare Geschwindigkeit des Gases in jedem Reaktor 47 cm/Sek oder weniger beträgt.

11. Verfahren nach Anspruch 2, wobei das Reaktionsprodukt durch den Wärmetauscher auf eine Temperatur von 0°C oder höher und 25°C oder niedriger gekühlt wird.

12. Verfahren nach Anspruch 10, wobei der Blasendurchmesser des in jeden der Reaktoren eingeführten Chlorgases 0,1 mm oder mehr und 5 mm oder weniger beträgt.

13. Verfahren nach Anspruch 1, wobei die Reaktion von jedem der Reaktoren in Gegenwart eines Hauptkatalysators und eines Cokatalysators durchgeführt wird, vorzugsweise,
wobei der Hauptkatalysator mindestens einer ist, ausgewählt aus der Gruppe, bestehend aus FeCl₃, Ferrocen, PtO₂, SbCl₃ und Fe, und/oder
der Cokatalysator mindestens einer ist, ausgewählt aus der Gruppe, bestehend aus S₂Cl₂, Thianthren, Diphenylselenid, Tetrachlorphenoxathin, Dichlorothiantren, Tetrachlorothiantren und Polychlorothiantren.

14. Verfahren nach Anspruch 13, wobei die Gehalte des Hauptkatalysators und des Cokatalysators (Hauptkatalysator : Cokatalysator) 1:0,59 oder mehr und 1:0,76 oder weniger, bezogen auf ein Molverhältnis, betragen.

## Revendications

1. Procédé de chloration d'un composé aromatique qui est le toluène, le procédé comprenant :
dans une pluralité de réacteurs de type colonne connectés en série, l'introduction d'un produit de réaction produit dans un premier réacteur dans un second réacteur,
l'introduction d'un chlore gazeux dans une partie basse de chaque réacteur dans la même quantité pour effectuer une réaction de chloration dans chaque réacteur, et
la décharge d'un gaz de chlorure d'hydrogène produit dans chacun des réacteurs depuis chaque réacteur.

2. Procédé selon la revendication 1, dans lequel le produit de réaction produit dans le premier réacteur est refroidi par un échangeur de chaleur disposé entre les réacteurs adjacents et introduit dans une partie basse du second réacteur de manière quantitative.

3. Procédé selon la revendication 1, dans lequel le gaz de chlorure d'hydrogène est également déchargé du produit de réaction produit dans le premier réacteur par un tambour tampon disposé entre les réacteurs adjacents et puis le produit de réaction est fourni au second réacteur.

4. Procédé selon la revendication 1, dans lequel le chlore gazeux est introduit dans une partie basse dans le réacteur à travers un diffuseur de gaz qui est disposé dans chaque réacteur et sur lequel sont formés une pluralité de trous.

5. Procédé selon la revendication 4, dans lequel un diamètre du trou du diffuseur de gaz est de 1 à 5 mm.

6. Procédé selon la revendication 1, dans lequel le chlore gazeux est introduit à une vitesse linéaire de 5 à 10 m/s à travers le trou du diffuseur de gaz.

7. Procédé selon la revendication 1, dans lequel le composé aromatique et le chlore gazeux sont introduits à raison de 1:1/16 à 1/8 sur la base des moles totales.

8. Procédé selon la revendication 7, dans lequel le taux de conversion dans chaque réacteur est de 1 à 15 **%.**

9. Procédé selon la revendication 4, dans lequel le composé aromatique est introduit dans la partie inférieure du réacteur à travers un diffuseur de liquide qui est disposé dans une partie inférieure à celle du diffuseur de gaz dans le réacteur et a une pluralité de trous de buse d'alimentation en matière première formés sur celui-ci, et
le composé aromatique est introduit à une vitesse linéaire de 10 à 40 m/sec à travers la buse d'alimentation en matière première.

10. Procédé selon la revendication 1, dans lequel la vitesse linéaire du gaz dans chaque réacteur est de 47 cm/s ou moins.

11. Procédé selon la revendication 2, dans lequel le produit de réaction est refroidi à une température de 0 °C ou plus et de 25 °C ou moins par l'échangeur de chaleur.

12. Procédé selon la revendication 10, dans lequel le diamètre de bulle du chlore gazeux introduit dans chacun des réacteurs est de 0,1 mm ou plus et de 5 mm ou moins.

13. Procédé selon la revendication 1, dans lequel la réaction de chacun des réacteurs est effectuée en présence d'un catalyseur principal et d'un cocatalyseur, de préférence
dans lequel le catalyseur principal est au moins un catalyseur sélectionné dans le groupe constitué de FeCh, de ferrocène, de PtO₂, de SbCl₃ et de Fe, et/ou
le cocatalyseur est au moins un cocatalyseur sélectionné dans le groupe constitué de S₂Cl₂, de thianthrène, de séléniure de diphényle, de tétrachlorophénoxathine, de dichlorothiantrène, de tétrachlorothiantrène, et de polychlorothiantrène.

14. Procédé selon la revendication 13, dans lequel les teneurs du catalyseur principal et du cocatalyseur (catalyseur principal : cocatalyseur) sont de 1:0,59 ou plus et de 1:0,76 ou moins sur la base d'un rapport molaire.
